# EUROPEAN PATENT SPECIFICATION

(11) **EP 0 944 407 B1**
(45) Date of publication and mention of the grant of the patent: **17.09.2003**
(21) Application number: 97913589.4
(22) Date of filing: 06.11.1997
(51) Int. Cl.: A61M 16/01

(54) **A DEVICE AND METHOD FOR RECOVERING ANAESTHETIC DURING THE USE OF INHALED ANAESTHETICS**
VORRICHTUNG UND VERFAHREN ZUM RÜCKGEWINNENVON NARKOSEMITTEL WÄHRENDDES INHALIERENS VON NARKOSEMITTELN
DISPOSITIF ET PROCEDE PERMETTANT DE RECUPERER UN ANESTHESIANT LORS DE L'UTILISATION D'ANESTHESIANTS PAR INHALATION

(30) Priority: 14.11.1996 SE 9604170
(43) Date of publication of application: 29.09.1999
(73) Proprietor: LOUIS GIBECK AB, 194 27 Upplands Väsby (SE)
(72) Inventor: LAMBERT, Hans, S-113 51 Stockholm (SE)
(74) Representative: Karlsson, Leif Karl Gunnar
(86) International application number: SE9701861
(87) International publication number: WO98020926

(56) References cited:
- EP-A- 0 496 336
- WO-A-88/07876

## Description

The present invention relates in a first aspect to a device of the type described in the preamble to claim 1, and in a second aspect to a method of the type described in the preamble to claim 4.

Such devices and methods are disclosed in EP-A-0 496 336, SEB 459 155 and SE-B-515 217, for instance. The purpose of such a device is to reduce the consumption of anaesthetic preparations when treating a patient, since preparations such as Fluothane-(2-bromine-2-chlorine-1,1,1-trifluorethane) are often relatively expensive. According to older, convertional technology the anaesthetic preparation is conducted away to the surroundings together with the air exhaled and is therefore lost, as well as contaminating the surroundings.

However, these devices are unable to compensate the loss of moisture upon exhalation. It would also be desirable to be able to improve the reflection efficiency, i.e. the proportion of exhaled anaesthetic which Is absorbed and returned to the gas inhaled.

Against this background, the object of the present invention is to alleviate this deficiency in the known devices and methods, and to increase the reflection efficiency.

According to a first aspect of the invention this has been achieved by providing a device of the type described in the preamble to claim 1 with the features defined in the characterizing part of this claim, and according to a second aspect thereof by providing a method of the type described in the preamble to claim 4 with the special measures defined in the characterizing part of this claim.

Thanks to the second unit with the ability to absorb and desorb water vapour, i.e. a moisture-heat exchanger, which is arranged in series with the first unit, i.e. the anaesthetic reflector, both anaesthetic and water vapour in the gas exhaled will be recovered since each component is absorbed in a separate unit. During inhaling, the anaesthetic preparation and water vapour recovered are released and desorbed out in the gases breathed, which will thus re-use a part of the anaesthetic preparation, as well as having part of its moisture content restored. The use of this device and this method thus enables a lower consumption of anaesthetic preparation than has been possible with known technology, as well as eliminating the need for any special supply of water vapour in order to avoid the risk of dehydration.

In a particularly preferred embodiment of the invention the second unit is situated between the patient and the first unit.

This has been found to have the surprising effect of greatly increasing the proportion of anaesthetic preparation recovered. It has been ascertained that the reflection efficiency is considerably increased with such an arrangement in comparison with other alternatives and even in comparison with a device without any moisture-heat exchanger at all. The optimal efficiency obtained with this preferred embodiment is probably a result of the reduced moisture content in the gas exhaled before it enters the anaesthetic reflector, increasing the ability of the latter to absorb and desorb anaesthetic. A greater proportion of this will therefore be returned to the patient in the gas inhaled.

In another preferred embodiment, preferably a variation of that described above, both the units, i.e. the anaesthetic reflector and the moisture-heat exchanger, are located in a common housing. This simplifies the construction, making it more compact and easily managed. It also ensures that the two units are compatible from the point of view of size.

The supply means for the anaesthetic preparation is preferably arranged in the tube between the patient and the air-heat exchanger, which offers maximum utilization of the anaesthetic preparation as well as more reliable control of dosages. In this case the means suitably comprises an vaporizer arranged in the patient tube. The advantages of such an arrangement are further described in patent application SEB-459 155 mentioned above.

The above and other advantageous embodiments of the device according to the invention are defined in the sub-claims to claim 1.

Equivalent advantageous embodiments of the method according to the invention are defined in the sub-claims to claim 4.

The invention is described in more detail in the following description of preferred embodiments with reference to the accompanying drawings, in which
Figure 1 illustrates schematically a reflector according to known technology,
Figure 2 illustrates schematically a preferred embodiment of the present invention, and
Figure 3 illustrates schematically a second embodiment of the present invention.

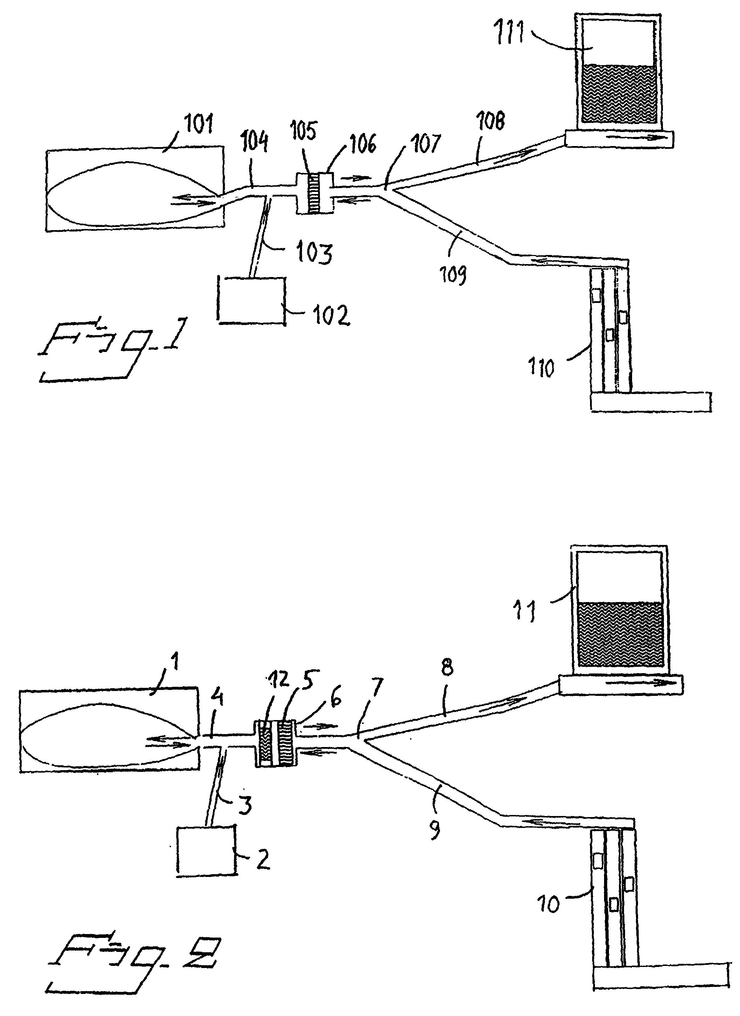

In Figure 1, which illustrates the principle for recovering anaesthetic according to known technology, 101 denotes a patient being treated with anaesthetic or an apparatus simulating the respiratory organs of a patient. The patient 101 is connected via the patient tube 104 to an anaesthetic reflector 105 arranged in a housing 106. A supply tube 103 for anaesthetic, connected to a supply 102 of anaesthetic, opens into the patient tube 104. The side of the reflector 105 facing away from the patient is connected via a Y-connection 107 to an outlet tube 108 for exhaled gas and an inlet tube 109 for inhaled gas, these being connected to a gas pump 111 and a gas supply device 110, respectively.

The reflector 105 consists of a material having the ability to absorb or desorb anaesthetic, e.g. woven active carbon. Measurements indicate that such a device is able to return 70% of the anaesthetic exhaled.

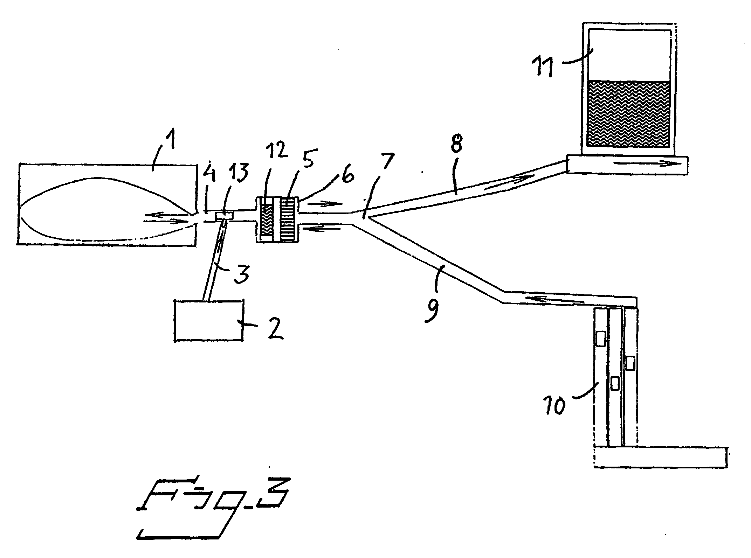

The device according to the invention illustrated in an embodiment shown in Figure 2 is constructed in an equivalent manner to that shown in Figure 1, with the difference that a moisture-heat exchanger 12 which absorbs and desorbs the moisture in the gases breathed is arranged in the housing 6 on the side of the anaesthetic reflector facing the patient. Thanks to this arrangement the proportion of anaesthetic recovered is increased to 80%. At the same time about 80% of the water vapour exhaled is also returned to the patient. As is clear from the Figure, the reflector 6 has a larger cross-sectional area than the moisture-heat exchanger, which has proved to be advantageous.

In Figure 2 both the moisture-heat exchanger 12 and the anaesthetic reflector 5 are shaped as a cylindrical plate with axial through-flow. However, it will be understood that the shape is of minor significance. One or both of these units may be annular in shape, for instance, with radial through-flow direction, in which case they may be arranged radially one outside the other.

The device according to Figure 3 differs from that in Figure 2 in that the anaesthetic preparation is supplied in liquid form through the tube 3 to the patient tube 4 and is only then vaporized in the patient tube via an anaesthetic vaporizer 13. This may be of a type described in more detail in the Swedish patent application No. 9601028-5 and offers certain advantages which are described in said patent application.

## Claims

1. A device for recovering anaesthetic during the use of inhaled anaesthetics, said device being connectable to a patient via a patient tube (4), and comprising a first unit (5) capable of absorbing and desorbing anaesthetic preparations, **characterized by** a second unit (12) arranged in series with the first unit (5) between the first unit (5) and the patient tube (4) and being capable of absorbing and desorbing water vapour, wherein a supply means (3) for anaesthetic preparations is connected to the patient tube (4) between said second unit (12) and the patient (1), said supply means (3) comprising an anaesthetic vaporizer (13) arranged in the patient tube (4).

2. A device as claimed in claim 1, wherein said first unit (5) and said second unit (12) are arranged in a common housing (6).

3. A device as claimed in claims 1 or 2, wherein said first unit (5) has a larger area transverse to the direction of flow of the gas than said second unit (12).

4. A method of recovering anaesthetic during the use of inhaled anaesthetics, wherein the gases inhaled and exhaled by a patient are caused to pass a unit for absorption of anaesthetic in the gas exhaled and desorption of anaesthetic to the gas inhaled, **characterized in that** the gases breathed are also caused to pass a unit for absorption of water vapour in the gases exhaled and desorption of water vapour in the gases inhaled, and wherein anaesthetic is supplied in the gas to be inhaled in a patient tube between said units and the patient, which anaesthetic is supplied in liquid form to the patient tube by a connector tube and is caused to vaporize therein by means of an anaesthetic vaporizer in the patient tube.

5. A method as claimed in claim 4, wherein water vapour in the gases exhaled is absorbed before anaesthetic in the gas exhaled is absorbed, and anaesthetic is desorbed to the gases inhaled before water vapour is desorbed to the gases inhaled.

## Patentansprüche

1. Vorrichtung zum Rückgewinnen von Narkosemittel während der Anwendung inhalierter Narkosemittel, welche Vorrichtung über einen Patientenschlauch (4) mit einem Patienten verbindbar ist und eine erste Einheit (5) aufweist, die in der Lage ist, Narkosemittel zu absorbieren und zu desorbieren, **gekennzeichnet durch** eine zweite Einheit (12), welche in Reihe mit der ersten Einheit (5) zwischen der ersten Einheit (5) und dem Patientenschlauch (4) angeordnet und in der Lage ist, Wasserdampf zu absorbieren und zu desorbieren, wobei eine Zuführeinrichtung (3) für Narkosemittel mit dem Patientenschlauch (4) zwischen der zweiten Einheit (12) und dem Patienten (1) verbunden ist, welche Zuführeinrichtung (3) einen in dem Patientenschlauch (4) angeordneten Narkosemittel-Verdampfer (13) aufweist.

2. Vorrichtung nach Anspruch 1, **dadurch gekennzeichnet, daß** die erste Einheit (5) und die zweite Einheit (12) in einem gemeinsamen Gehäuse (6) angeordnet sind.

3. Vorrichtung nach Anspruch 1 oder 2, **dadurch gekennzeichnet, daß** die erste Einheit (5) eine größere Fläche quer zur Strömungsrichtung des Gases als die zweite Einheit (12) aufweist.

4. Verfahren zum Rückgewinnen von Narkosemittel während der Anwendung inhalierter Narkosemittel, bei welchem die von einem Patienten inhalierten und ausgeatmeten Gase über eine Einheit zum Absorbieren von Narkosemittel in dem ausgeatmeten Gas und zum Desorbieren von Narkosemittel in das inhalierte Gas geleitet werden, **dadurch gekennzeichnet, daß** die geatmeten Gase auch eine Einheit zum Absorbieren von Wasserdampf in den ausgeatmeten Gasen und zum Desorbieren von Wasserdampf in die inhalierten Gase passieren, wobei das Narkosemittel in das zu inhalierende Gas in einen Patientenschlauch zwischen den Einheiten und dem Patienten zugeführt wird welches Narkosemittel in flüssiger Form mittels eines Verbindungsschlauchs zu dem Patientenschlauch zugeführt wird und in dem Patientenschlauch mittels eines Narkosemittel-Verdampfers verdampft wird.

5. Verfahren nach Anspruch 4, **dadurch gekennzeichnet, daß** der Wasserdampf in den ausgeatmeten Gasen absorbiert wird, bevor das Narkosemittel in dem ausgeatmeten Gas absorbiert wird und daß das Narkosemittel in die zu inhalierenden Gase desorbiert wird, bevor der Wasserdampf in die inhalierten Gase desorbiert wird.

## Revendications

1. Dispositif pour la récupération d'anesthésiant pendant l'utilisation d'anesthésiants inhalés, ledit dispositif pouvant être relié à un patient au moyen d'un tube du patient (4), et comprenant une première unité (5) capable d'absorber et de désorber des préparations anesthésiantes, **caractérisé par** une seconde unité (12) montée en série avec la première unité (5) entre la première unité (5) et le tube du patient (4) et étant capable d'absorber et de désorber de la vapeur d'eau, dans lequel un moyen d'alimentation (3) en préparations anesthésiantes est raccordé au tube du patient (4) entre ladite seconde unité (12) et le patient (1), ledit moyen d'alimentation (3) comprenant un vaporisateur d'anesthésiant (13) disposé dans le tube du patient (4).

2. Dispositif selon la revendication 1, dans lequel ladite première unité (5) et ladite seconde unité (12) sont montées dans un bottier commun (6).

3. Dispositif selon les revendications 1 et 2, dans lequel la superficie de ladite première unité (5) transversale à la direction du flux de gaz est supérieure à celle de ladite seconde unité (12).

4. Procédé de récupération d'anesthésiant pendant l'utilisation d'anesthésiants inhalés, dans lequel les gaz inhalés et exhalés par un patient sont dirigés dans une unité d'absorption d'anesthésiant contenu dans le gaz exhalé et de désorption d'anesthésiant contenu dans le gaz inhalé, **caractérisé en ce que** les gaz respirés sont aussi dirigés dans une unité d'absorption de vapeur d'eau contenue dans les gaz exhalés et de désorption de vapeur d'eau contenue dans les gaz inhalés, et dans lequel l'anesthésiant est amené dans le gaz à inhaler dans un tube du patient entre lesdites unités et le patient, ledit anesthésiant est fourni sous forme liquide dans le tube du patient par un tube de raccordement, et est vaporisé dans celui-ci au moyen d'un vaporisateur d'anesthésiant dans le tube du patient.

5. Procédé selon la revendication 4, dans lequel la vapeur d'eau contenue dans les gaz exhalés est absorbée avant que l'anesthésiant contenu dans le gaz exhalé soit absorbé, et dans lequel l'anesthésiant est désorbé dans les gaz inhalés avant que la vapeur d'eau ne soit désorbée dans les gaz inhalés.
